# EUROPÄISCHE PATENTSCHRIFT

(11) **EP 2 473 856 B1**
(45) Veröffentlichungstag und Bekanntmachung des Hinweises auf die Patenterteilung: **22.03.2017**
(21) Anmeldenummer: 09737329.4
(22) Anmeldetag: 03.09.2009
(51) Int. Cl.: G01N 33/86

(54) **VERFAHREN ZUR BESTIMMUNG DER QUALITÄT VON HEPARIN**
METHOD FOR DETERMINING THE QUALITY OF HEPARIN
PROCÉDÉ DE DÉTERMINATION DE LA QUALITÉ DE L'HÉPARINE

(43) Veröffentlichungstag der Anmeldung: 11.07.2012
(73) Patentinhaber: Christian-Albrechts-Universität zu Kiel, 24118 Kiel (DE)
(72) Erfinder: ALBAN, Susanne, 24146 Kiel (DE); LÜHN, Susanne, 24118 Kiel (DE); SCHIEMANN, Simone, 49082 Osnabrück (DE)
(74) Vertreter: Müller Fottner Steinecke
(86) Internationale Anmeldenummer: PCT/DE2009/001215
(87) Internationale Veröffentlichungsnummer: WO 2011/026450

(56) Entgegenhaltungen:
- JAGT RICHARD B C ET AL: "Pattern-based recognition of heparin contaminants by an array of self-assembling fluorescent receptors." ANGEWANDTE CHEMIE (INTERNATIONAL ED. IN ENGLISH) 2009, Bd. 48, Nr. 11, 4. Februar 2009 (2009-02-04), Seiten 1995-1997, XP002564921 ISSN: 1521-3773
- CLARK M ET AL: "Molecular profiling of heparinase-I resistant glycosaminoglycans in contaminated heparins. Comparative studies with uncontaminated heparin and porcine oversulfated chondroitin sulfate" INTERNATIONAL ANGIOLOGY, TORINO, IT, Bd. 27, Nr. 5, 1. Oktober 2008 (2008-10-01), Seiten 370-376, XP009127715 ISSN: 0392-9590
- WANG SHENLIANG ET AL: "Discovery of heparin chemosensors through diversity oriented fluorescence library approach." CHEMICAL COMMUNICATIONS (CAMBRIDGE, ENGLAND) 14 MAR 2008, Nr. 10, 14. März 2008 (2008-03-14), Seiten 1173-1175, XP002564922 ISSN: 1359-7345
- ALBAN SUSANNE ET AL: "Prothrombin time for detection of contaminated heparins." THE NEW ENGLAND JOURNAL OF MEDICINE 18 DEC 2008, Bd. 359, Nr. 25, 18. Dezember 2008 (2008-12-18), Seiten 2732-2734, XP002564923 ISSN: 1533-4406
- LIU HAIYING ET AL: "Lessons learned from the contamination of heparin" NATURAL PRODUCT REPORTS, ROYAL SOCIETY OF CHEMISTRY, GB, Bd. 26, Nr. 3, 1. März 2009 (2009-03-01), Seiten 313-321, XP009127837 ISSN: 0265-0568

## Beschreibung

Die Erfindung betrifft ein Verfahren zur Bestimmung von Verunreinigungen von Heparin mit anderen sulfatierten Glykanen. Insbesondere betrifft die Erfindung ein Verfahren zur Bestimmung von unzulässigen Verunreinigungen von Heparin, insbesondere Heparinimitate wie übersulfatiertes Chondroitinsulfat (OSCS) und darüber hinaus hohe Gehalte an anderen Glykosaminoglykanen (GAG) und Proteinen. Das Verfahren kann auf Heparin im Sinne von Arzneistoff Heparin (API, active pharmaceutical ingredient) als auch in Sinne von Heparinzubereitungen und Fertigarzneimittel angewendet werden.

Wie bekannt sind Heparine Arzneimittel zur Therapie und Prophylaxe thromboembolischer Erkrankungen. Es handelt sich um Arzneistoffe tierischen Ursprungs. Rohheparin wird heute ausschließlich aus Schweinedarmmukosa isoliert. Es besteht aus komplexen Mischungen hochsulfatierter Glykosaminoglykane (GAG), die zusätzlich erheblichen Schwankungen in ihrer individuellen Zusammensetzung unterliegen. Neben Heparin können auch die Glykosaminoglykane (GAG) Chondroitinsulfat und Dermatansulfat in geringen Mengen enthalten sein.

Durch Aufreinigung von Rohheparin erhält man den Arzneistoff Heparin, d.h. unfraktioniertes Heparin (UFH). Aus UFH werden durch Degradation verschiedene niedermolekulare Heparine (NMH) hergestellt, die zu mindestens 60 % (m/m) aus Molekülen mit einem Molekulargewicht < 8.000 bestehen.

Ab November 2007 kam es in den USA zu einem drastischen Anstieg von schwerwiegenden unerwünschten Arzneimittelwirkungen (UAW) wie starkem Blutdruckabfall und anaphylaktoiden Reaktionen sowie Todesfällen bei mit UFH behandelten Patienten. Auch in Deutschland wurden bis Anfang März 2008 31 Fälle unerwünschter Arzneimittelwirkungen (UAW) gemeldet, die auf das eingesetzte Heparinpräparat zurückzuführen waren.

Im Februar 2008 wurde bekannt, dass die die unerwünschten Arzneimittelwirkungen (UAW) auslösenden Heparinchargen aus China kamen. Im März 2008 entdeckte man darin eine Kontamination, die schließlich als übersulfatiertes Chondroitinsulfat (OSCS) identifiziert wurde und in einer Höhe von 5 bis 50 % enthalten war. Darüber hinaus enthielten nicht nur zahlreiche unfraktionierte Heparin (UFH)-Produkte kontaminiertes, d.h. mit übersulfatiertem Chondroitinsulfat (OSCS) verunreinigtes Heparin, sondern auch niedermolekulare Heparine (NMH).

Zwar weist übersulfatiertes Chondroitinsulfat (OSCS) als polysulfatiertes Glykosaminoglykan eine ähnliche chemische Struktur und eine ähnliche biologische Aktivität wie Heparin auf. Allerdings ist auch bekannt, dass stark sulfatierte Polysaccharide, wie übersulfatierte Chondroitine (OSCS), noch vielfältige zusätzliche Effekte haben. Unter anderem aktivieren sie das Kontaktsystem und bewirken so die Freisetzung von Bradykinin, einem starken Vasodilatator, sowie die Bildung von Anaphylatoxinen. Diese Befunde stützen den oben genannten Befund, dass die oben genannten unerwünschten Arzneimittelwirkungen (UAW) der mit übersulfatiertem Chondroitinsulfat (OSCS) kontaminierten Heparinpräparate auf das übersulfatierte Chondroitinsulfat (OSCS) zurückzuführen sind.

Fatalerweise war weder mit den Reinheits- und Identitätsprüfungen der Monographien zu unfraktioniertem Heparin (UFH) und niedermolekularem Heparin (NMH) des Europäischen Arzneibuchs (Pharmacopoea Europaea, PhEur) sowie der Arzneibücher anderer Nationen wie z.B. die USP der USA, noch mit den Methoden zur Wertbestimmung eine Verunreinigung mit OSCS zu erkennen.

Der Wirkwert von unfraktioniertem Heparin (UFH) wird üblicherweise anhand der gerinnungshemmenden Wirkung in der aPTT (aktivierte partielle Thromboplastinzeit) bestimmt. Nach eigenen Untersuchungen mit übersulfatiertes Chondroitinsulfat (OSCS) und Heparin, welches mit übersulfatiertem Chondroitinsulfat (OSCS) gespikt wurde (1 - 52,2 %), konnte gezeigt werden, dass übersulfatiertes Chondroitinsulfat (OSCS) in der aPTT etwas weniger stark gerinnungshemmend wirkt als unfraktioniertes Heparin (UFH). Im Rahmen der Qualitätsprüfung von Heparin lässt sich allerdings selbst eine Kontamination von 20 % übersulfatiertes Chondroitinsulfat nicht feststellen, da wegen der natürlichen Chargenschwankungen des biogenen Wirkstoffes Heparin lediglich eine Mindestaktivität von 150 IU/mg gefordert wird.

Die Wertbestimmung der niedermolekularen Heparine (NMH) hingegen umfasst die Messung ihrer anti-Faktor Xa (aXa)- und anti-Thrombin (aIIa)-Aktivität. Da auch hier die Mindestaktivität mit 70 aXa- IU/mg gering und der für die einzelnen niedermolekularen Heparine (NMH) jeweils erlaubte Bereich breit ist, fällt eine Kontamination mit übersulfatiertem Chondroitinsulfat (OSCS), das keine aXa-Aktivität besitzt, nicht ins Gewicht. Die aIIa-Aktivität von übersulfatiertem Chondroitinsulfat (OSCS) wird mit dem Testsystem des Arzneibuches nicht erfasst. Auch andere potentielle Heparinverunreinigungen, z.B. Curdlan-, Dextran-, Pullulansulfate, würden sich mit den üblichen Gerinnungs- und chromogenen Tests nicht nachweisen lassen.

Da einerseits der Bedarf an Heparin seit Jahren sukzessive steigt, andererseits jedoch die natürlichen Ressourcen begrenzt sind und über 80 % des weltweit gehandelten Heparins aus China stammen, sind auch künftig Kontaminationen von Heparin mit OSCS oder auch anderen heparinähnlich wirkenden Substanzen nicht ausgeschlossen. Insbesondere ist der Umstand, dass Chondroitinsulfat, aus dem übersulfatiertes Chondroitinsulfat (OSCS) einfach herzustellen ist, um den Faktor 200 günstiger herzustellen ist als Heparin, für den Hersteller ein finanzieller Anreiz, der für den Patienten allerdings fatale Folgen haben kann.

Die qualitative und quantitative Bestimmung von Heparin ähnlichen sulfatierten Glykosaminoglykanen, insbesondere von übersulfatiertem Chondroitinsulfat (OSCS), als Heparin verunreinigende Kontaminanten erfolgt derzeit mittels ¹H-Kernresonanzspektroskopie (¹H-NMR) und Kapillarelektrophorese (CE).

Diese Verfahren sind jedoch apparativ aufwändig, zeitaufwändig, kostspielig und erfordern speziell geschultes Personal für die Qualitätssicherung von Heparin. Als Schnelltest im klinischen Labor sind sie völlig ungeeignet.

R. B. C. Jagt et al. (R. B. C. Jagt, R. F. Gómez-Biagi, M. Nitz. Pattern-Based Recognition of Heparin Contaminants by an Array of Self-Assembling Fluorescent Receptors. Angew. Chem. 2009 (48 (11)): 1995-1997) beschreibt ein Verfahren zur Bestimmung von Kontaminierungen von Heparin mittels eines Arrays aus selbstzusammenbauenden, supramolekularen Fluoreszenzrezeptoren, die bei Vorliegen sulfatierter Glykane ihre Fluoreszenzeigenschaften verändern. Das Signalprofil, das sich daraus ergibt, ist für Heparin und die diversen möglichen kontaminierenden sulfatierten Glykane jeweils verschieden, was eine Diskriminierung von Heparin und anderen kontaminierenden sulfatierten Glykanen erlaubt.

Aufgabe der Erfindung ist es daher ein Verfahren zu schaffen, das eine qualitative und bevorzugt quantitative Beurteilung von Heparin insbesondere im Hinblick auf Kontamination mit anderen sulfatierten Glykanen, insbesondere heparinähnlich wirkenden Substanzen, speziell übersulfatiertem Chondroitinsulfat (OSCS), ermöglicht.

Diese Aufgabe wird durch das Verfahren mit den Merkmalen von Anspruch 1 gelöst. Die Unteransprüche geben vorteilhafte Ausgestaltungen der Erfindung wieder.

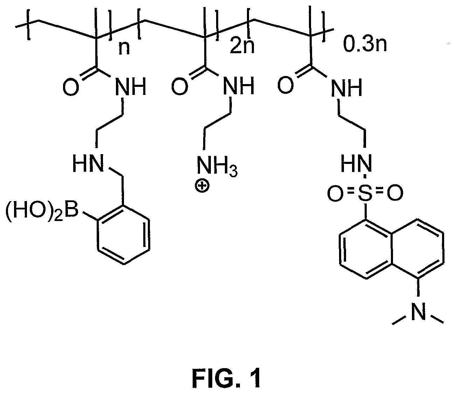

Ausgangspunkt der Erfindung ist die Verwendung eines an sich bekannten fluoreszierenden Heparinsensorpolymers (siehe W Sun, H Bandmann, T Schrader. A Fluorescent Polymeric Heparin Sensor. Chem Eur J. 2007 (13): 7701-7707). Dieses bekannte im Folgenden als Polymer-H bezeichnete Copolymer, dessen Strukturformel in Fig. 1 dargestellt ist, besteht aus drei Typen von funktionellen Monomeren, basierend auf einem Methacrylamidgerüst: (1) Dansylmonomere als Fluorophore, (2) ortho-Aminomethylphenylboronate zur Sulfat- und Glykolbindung und (3) Ethylammoniumeinheiten für zusätzliche elektrostatische Wechselwirkungen (Abb. 1). Sein mittleres hydrodynamische Volumen laut GelPermeationschromatographie (GPC) beträgt ~116.000 Da. Die Messung der Fluorszenzintensität erfolgt bei λₑₓ 320nm bzw. λₑₘ 510 nm.

Allerdings bindet das bekannte Heparinsensorpolymer (Fig. 1) nicht nur Heparin, sondern auch andere sulfatierte Glykane, insbesondere übersulfatiertes Chondroitinsulfat (OSCS), ohne dass es zu einer (apparenten) unterschiedlichen Ausprägung der Fluoreszenz des Indikatormoleküls käme. Allein mit dem Indikatormolekül kann also eine Kontamination von Heparin mit anderen sulfatierten Glykanen, insbesondere übersulfatiertes Chondroitinsulfat (OSCS), nicht festgestellt werden.

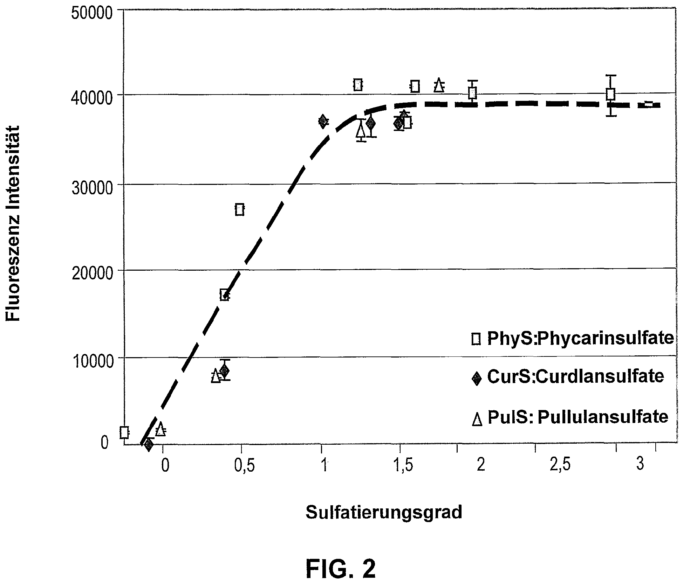

Es wurde herausgefunden, dass der einzige strukturelle Parameter der Glykane, der das Ausmaß der Fluoreszenzintensitätssteigerung signifikant beeinflusst, der Sulfatierungsgrad (DS, d.h. die durchschnittliche Zahl der Sulfatgruppen pro Monosaccharid) ist. Je höher dieser ist, desto stärker sind die Wechselwirkungen mit Polymer1 und desto größer ist die Fluoreszenzzunahme (Fig. 2). Diese Abhängigkeit zeigt sich bis zu einem DS von ∼1,4; höhere DS-Werte führen zu keiner weiteren Steigerung.

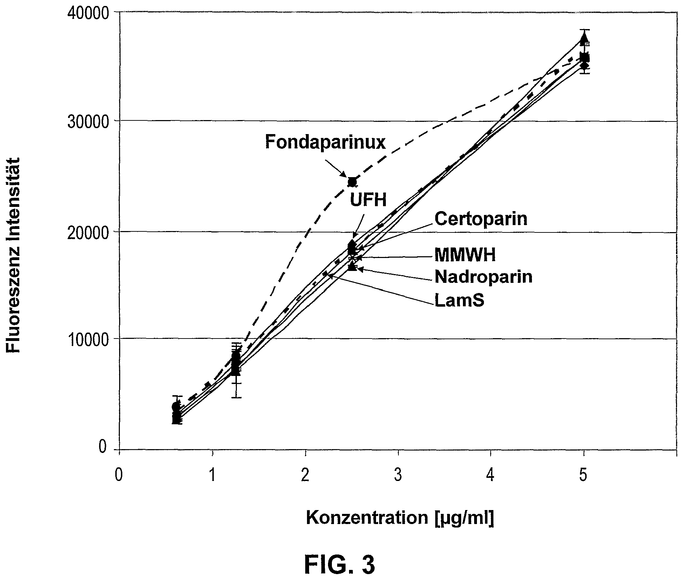

Da sich der Sulfatierungsgrad (DS) verschiedener Heparine nur geringfügig unterscheidet, lassen sich alle Heparine mit einer einzigen Kalibrierkurve quantifizieren (Fig. 3), womit der Polymer-Assay einen klaren Vorteil gegenüber den üblichen Heparintests bietet. Außerdem können mit diesem Test auch andere sulfatierte Glykane und somit sowohl OSCS und Dermatansulfat als auch andere potentielle Heparin-Kontaminationen quantitativ bestimmt werden (Fig. 3). So ist die Fluoreszenzsteigerung von Polymer-H durch Laminarinsulfat (LamS), einem selektiv, d.h. in Position 2 und 4, sulfatierten linearen β-1,3-Glucansulfat (Mr 11.500, DS 1,15), ähnlich wie die der Heparine.

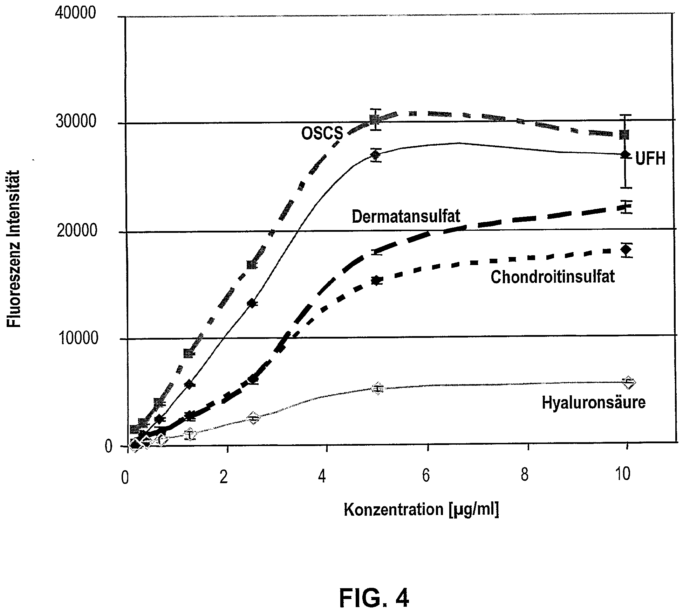

Die konzentrationsabhängigen Bindungskurven mehrerer sulfatierter Glykane an Polymer-H ist in Fig. 4 dargestellt.

Überraschenderweise konnte nun trotz der hohen Variabilität der Heparinmoleküle festgestellt werden, dass eine Inkubation von Heparin mit einem Heparin spezifisch spaltenden Enzym, besonders bevorzugt mit Heparinlyase I (EC 4.2.2.7), zu Spaltprodukten führt, die nicht mehr mit dem Heparinsensorpolymer als Indikatormolekül nachgewiesen werden können.

Durch Abbau des im Heparin vorhandenen Heparinanteils durch ein geeignetes spezifisch Heparin spaltendes Enzym ist es also ermöglicht, lediglich den Anteil an nicht abgebauten sulfatierten Glykanen mit dem Indikatormolekül nachzuweisen und so gegebenenfalls Kontamination des Heparin mit sulfatierten Glykanen, insbesondere mit übersulfatiertem Chondroitinsulfat (OSCS), festzustellen.

Voraussetzung hierfür ist, dass die eingesetzte Heparin spaltende Enzymmenge wenigstens derjenigen Menge entspricht, die eine in etwa identische Menge Kontaminanten freies Heparin vollständig abbauen würde. Der Fachmann wird also in Vorversuchen mit nicht-kontaminiertem Heparin mit geeigneten Verdünnungen des Heparin spaltenden Enzyms die minimale Menge Enzym bestimmen, bei der ein vollständiger Abbau des Heparins erfolgt.

Aus im Folgenden genannten Gründen ist der Einsatz der minimalen Enzymmenge, bei der ein vollständiger Abbau des Heparins erfolgt, besonders vorteilhaft.

Es wurde gefunden, dass sich der Abbau des Heparins sehr gut mit einem aXa-Test kontrollieren lässt, da abgebautes Heparin keine aXa-Aktvität mehr besitzt. Durch Variation der Heparin spaltenden Enzymmenge wurde mit Hilfe des aXa-Assays die Enzymmenge bestimmt, bei der ein vollständiger Abbau von Heparin eintritt.

Überraschenderweise wurde allerdings im aXa-Test auch beobachtet, dass bei einem absichtlich mit übersulfatiertem Chondroitinsulfat (OSCS) versetzten Heparin trotz vermeintlich vollständigem Abbau des Heparinanteils durch Heparinase eine auf Heparin zurückzuführende aXa-Aktivität im mit Heparinase behandelten Ansatz verblieben ist. Es kann daher angenommen werden, dass das übersulfatierte Chondroitinsulfat (OSCS) als Inhibitor der Heparinase fungiert und den Abbau von Heparin konzentrationsabhängig unterbindet.

Es wurde ferner im aXa-Test beobachtet, dass auch andere sulfatierte Glykane, die die Wirkung von Heparin imitieren wie z.B. Dextransulfat, die Heparinase hemmen. Im Gegensatz dazu wurde festgestellt, dass sulfatierte Glykane, die als natürliche Verunreinigungen in Heparin enthalten sein können, insbesondere Dermatansulfat und Chondroitinsulfat, die Heparinase nicht hemmen.

Im Fluoreszenztest wurde überraschenderweise festgestellt, dass nach Behandlung mit Heparinase eine Kontamination von Heparin mit anderen Heparin imitierenden sulfatierten Glykanen wie übersulfatiertem Chondroitinsulfat (OSCS) nachweisbar ist, während eine Kontamination mit Dermatan- oder Chondroitinsulfat nicht nachweisbar ist. Daher sind sulfatierte Glykane in Heparinimitate einerseits und natürliche Glykosaminoglykane (GAG) andererseits zu differenzieren.

Um die Enzymmenge im Vortest zu bestimmen, die einen vollständigen Abbau von Kontaminanten freiem Heparin bewirkt, wird daher ein Kontaminanten freies Standardheparin verwendet, das in einer festgelegten konstanten Menge eingesetzt wird (siehe oben). Von einem auf Kontaminanten zu überprüfenden Heparin wird ebenfalls diese Menge eingesetzt. Handelt es sich um ein Fertigarzneimittel, das üblicherweise nicht in Milligramm, sondern in Einheiten quantifiziert ist, wird die analog einzusetzende Menge an Einheiten üblicherweise aus den Herstellerangaben zur Heparinaktiviät (Einheiten pro Milligramm) errechnet.

Wird beispielsweise bei Durchführung des Verfahrens mit den Merkmalen von Anspruch 1 keine auf sulfatierte Glykane hinweisende Fluoreszenz detektiert und dennoch im aXa-Test eine aXa-Aktivität erfasst, so kann davon ausgegangen werden, dass im Heparin entweder Kontaminanten mit aXa-Aktivität (Faktor Xa-Inhibitoren) oder andere die Aktivität des Heparin abbauenden Enzyms inhibierenden Kontaminanten vorhanden sind.

Ist andererseits bei Durchführung des Verfahrens mit den Merkmalen von Anspruch 1 eine auf sulfatierte Glykane hinweisende Fluoreszenz detektiert und stellt sich im aXa-Test heraus, dass keine aXa-Aktivität im mit dem Enzym inkubierten Heparinvorhanden ist, muss die Fluoreszenz auf andere Verunreinigungen, z.B. Proteine oder extrem hohen Beimengungen an natürlichen Glykosaminoglykanen (GAG), zurückzuführen sein, nicht jedoch auf Heparinimitate wie übersulfatiertes Chondroitinsulfat (OSCS).

Ein besonderer Vorteil bei Durchführung des Verfahrens mit den Merkmalen von Anspruch 1 liegt in der Inkubation des Heparins mit einer minimalen Menge Heparinase, die bei Fehlen von Heparinase inhibierenden Kontaminanten zu einem vollständigen Abbau des im Heparin vorhandenen Heparinanteils führten würde. Denn durch die inhibierende Wirkung von Heparinimitaten wie OSCS auf die Heparinase können selbst solch geringe Mengen OSCS, die allein nicht mit dem Polymertest nachweisbar wären, indirekt durch das Vorhandensein von nicht abgebautem Heparin nachgewiesen werden.

Die größte Empfindlichkeit, die inhibierende Wirkung der Heparinimitate nachzuweisen, und damit ein besonderer Vorteil ist gegeben, wenn gerade so viel Enzym eingesetzt wird, wie bei nicht-kontaminiertem Heparin notwendig wäre, um einen vollständigen Abbau des Heparins zu erreichen.

Wird bei Durchführung des Verfahrens mit den Merkmalen von Anspruch 1 eine auf Heparinimitate hinweisende Fluoreszenz detektiert und stellt sich im aXa-Test heraus, dass auch eine aXa-Aktivität im mit dem Enzym inkubierten Heparin vorhanden ist, ist das Vorhandensein von Heparinimitat eindeutig nachgewiesen. Hierbei wird die Summe aller vorhandenen Heparinimitate erfasst. Daraus ergibt sich der Vorteil, dass auch eine absichtlich verwendetete Mischung verschiedener Heparinimitate, die den Einzelnachweis erschweren soll, nachweisbar ist.

Es versteht sich, dass zur Gewährleistung überprüfbarer Messergebnisse parallel eine Standardprobe mit ausschließlich einer vorbestimmten Menge an Kontaminanten freiem Heparin angesetzt wird. Sie wird ohne und mit vorheriger Inkubation mit dem spezifisch Heparin spaltenden Enzym im aXa-Test und Fluoreszenztest vermessen, um die Aktivität des Enzyms überprüfen zu können. Zusätzlich sollten die Heparinproben auch ohne vorherige Inkubation im aXa-Test und Fluoreszenztest vermessen werden. Dadurch kann im aXa-Test festgestellt werden, ob die Probe überhaupt vollständig oder überwiegend aus Heparin besteht, und im Fluoreszenztest sind andere Verunreinigungen, die selbst fluoreszieren oder die Fluoreszenzintensität des Indikatormoleküls beeinflussen, nachweisbar. Diese Ansätze sollten eindeutige Ergebnisse liefern.

Schließlich ist es möglich, Kalibrierungskurven von Heparin mit steigenden Mengen übersulfatierten Chondroitinsulfats (OSCS) oder einem anderen Heparinimitat anzufertigen, wobei ein (unter Umständen erst bei geeigneter Verdünnung) Messwert eines Heparins, das mit übersulfatiertem Chondroitinsulfat (OSCS) oder dem anderen Heparinimitat kontaminiert ist, den Kalibrierungswerten direkt zugeordnet und so der Grad der Kontamination angegeben werden kann. Da eine Verunreinigung mit jeglichen Heparinimitaten inakzeptabel ist, kann auch allgemein eine Kalibrierkurve von Heparin mit steigenden Mengen übersulfatierten Chondroitinsulfats (OSCS) verwendet werden und der Grad der Kontamination als OSCS-Äquivalent angegeben werden.

Die Erfindung wird anhand eines bevorzugt ausgestalteten Ausführungsbeispiels und der beigefügten Zeichnungen näher erläutert. Diese zeigen:
- Fig. 1: zeigt die allgemeine Strukturformel des als Indikatormolekül verwendeten Polymer-H
- Fig. 2: zeigt die Abhängigkeit der Fluoreszenzintensität von Polymer-H vom Sulfatierungsgrad (DS) sulfatierter Glykane. Mit zunehmender Ladungsdichte der sulfatierten Glykane verstärken sich die Wechselwirkungen mit Polymer-H und damit die Fluoreszenzintensität. Ab einem DS von ∼1,4 kommt es zu keiner weiteren Fluoreszenzzunahme.
- Fig. 3: zeigt die Steigerung der Fluoreszenzintensität von Polymer-H durch verschiedene Heparine und Laminarinsulfat. Alle untersuchten Heparine bewirken die gleiche konzentrationsabhängige Fluoreszenzzunahme, so dass nur noch eine Standardreihe für ihre Quantifizierung nötig ist.
- Fig. 4: zeigt die Steigerung der Fluoreszenzintensität von Polymer-H durch übersulfatiertes Chondroitinsulfat (OSCS) und verschiedene Glykosaminoglykane. Die Substanzen differieren in ihrem Sulfatierungsgrad (DS) und führen daher zu einer unterschiedlich stark ausgeprägten Fluoreszenzzunahme.

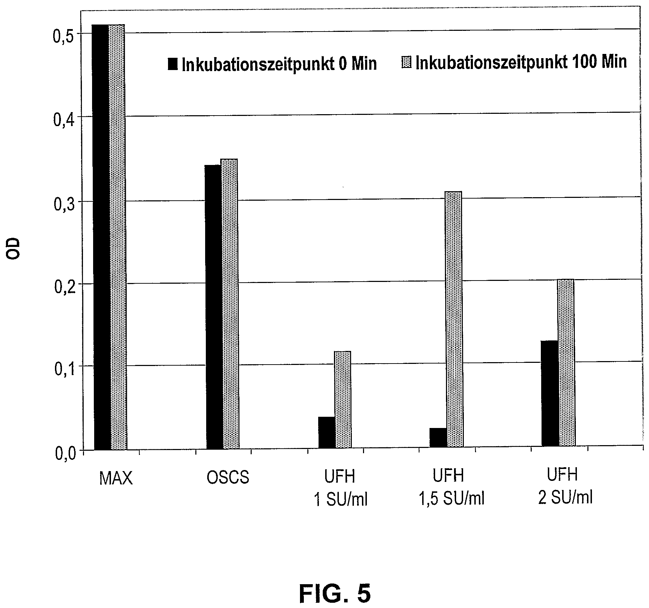

- Fig. 5: zeigt die anti-Faktor-Xa (aXa) Aktivität von Heparin zu Beginn und nach 100 min Inkubation mit Heparinase. Die angegebenen Konzentrationen entsprechen den jeweiligen Pipettierkonzentrationen; die Heparinkonzentration im Ansatz lag somit bei 10 µg/ml. OSCS besitzt keine aXa-Aktivität, die "optical density" (OD) entspricht ungefähr der der ungehemmten FXa-Aktivität (Max-Wert). Bei Verwendung von 2,5 SU/ml Heparinase (Pipettierkonzentration) beginnt der Heparinabbau sofort, so dass bereits beim 0 min-Wert (nach 1 min Schütteln) die aXa-Aktivität deutlich reduziert ist. Eine Konzentration von 1,5 SU/ml ergab reproduzierbar einen nahezu vollständigen Abbau und insgesamt bessere Resultate als 1,0 SU/ml.

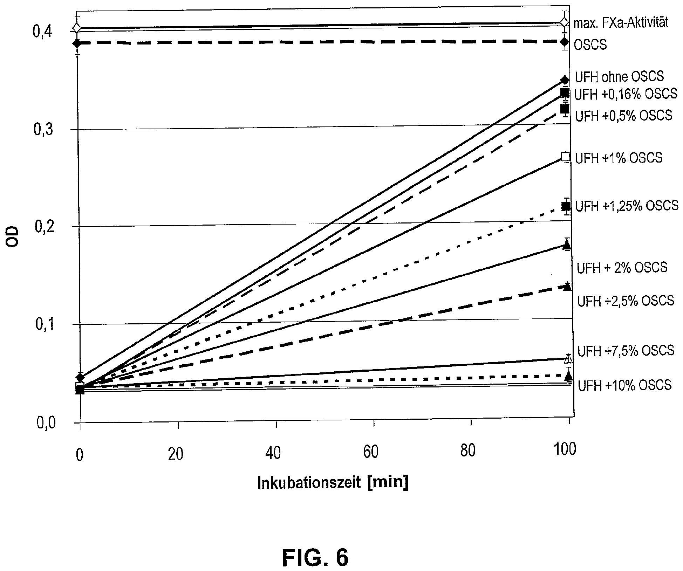

- Fig. 6: zeigt die aXa-Assay-Ergebnisse von UFH-OSCS-Mischungen zu Beginn und nach 100 min Inkubation mit 1,5 SU/ml Heparinase. Die OD von OSCS bleibt gleich und entspricht ungefähr der des MAX-Wertes, d.h. keine Hemmung der FXa-Aktivität. Die aXa-Aktivität von UFH zu Beginn wird durch den enzymatischen Abbau fast vollständig zerstört, die von UFH, das mit OSCS kontaminiert ist, jedoch nur partiell.

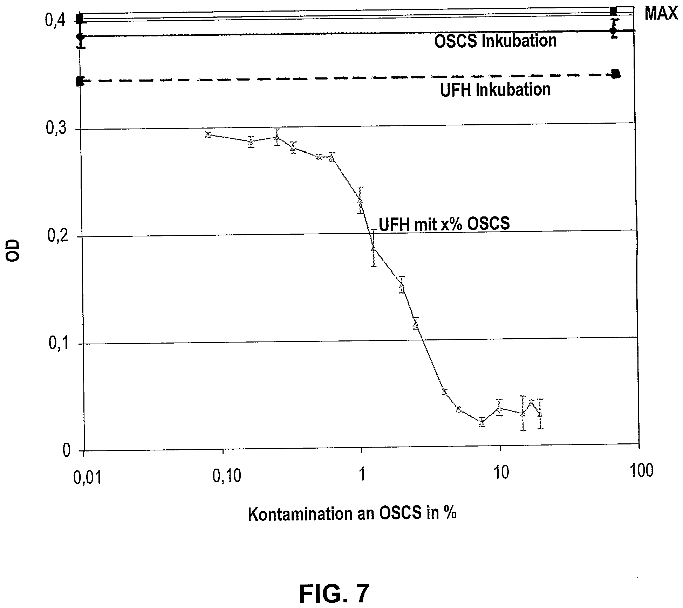

- Fig. 7: zeigt die aXa-Assay-Ergebnisse von OSCS-Heparin-Mischungen nach Inkubation (100 min) mit 1,5 SU/ml Heparinase. Abnahme der OD und somit Zunahme der aXa-Aktivität in Abhängigkeit von der prozentualen Kontamination des Heparins mit OSCS. Da OSCS selbst keine aXa-Aktivität (OD-MAX) besitzt, zeigt dies die konzentrationsabhängige Hemmung des enzymatischen Abbaus von UFH.

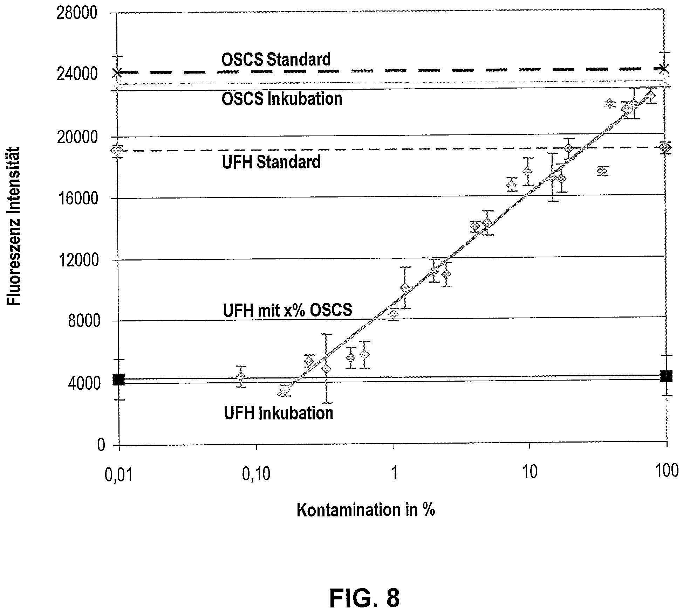

- Fig. 8: zeigt die Fluoreszenztest-Ergebnisse von OSCS-Heparin-Mischungen nach Inkubation (100 min) mit 1,5 SU/ml Heparinase. Zunahme der Fluoreszenzintensität in Abhängigkeit von der prozentualen Kontamination des Heparins mit OSCS (R² = 0,97).

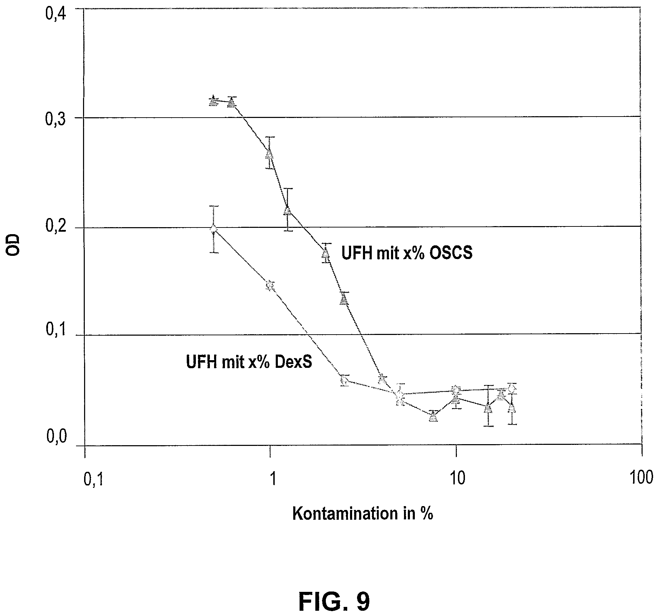

- Fig. 9: zeigt aXa-Assay-Ergebnisse von Dextransulfat-Heparin-Mischungen im Vergleich zu OSCS-Heparin-Mischungen nach Inkubation (100 min) mit 1,5 SU/ml Heparinase.

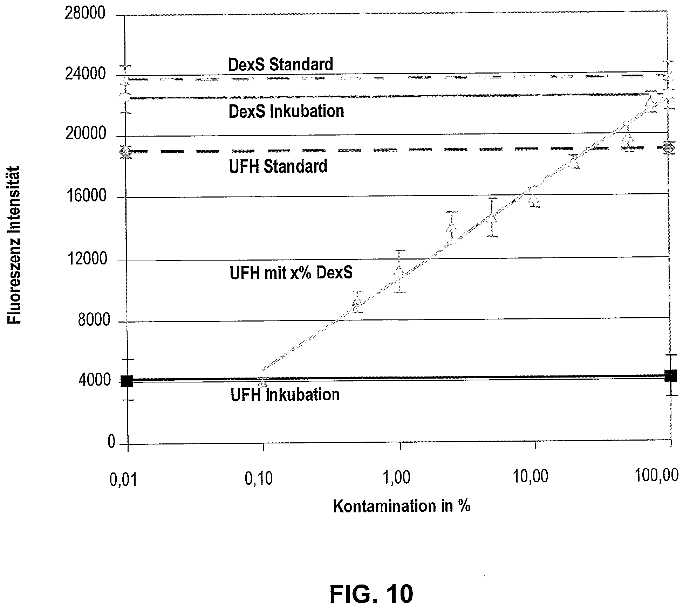

- Fig. 10: zeigt Fluoreszenztest-Ergebnisse von Dextransulfat-Heparin-Mischungen nach Inkubation (100 min) mit 1,5 SU/ml Heparinase. Zunahme der Fluoreszenzintensität in Abhängigkeit von der prozentualen Kontamination des Heparins mit DexS (R² = 0,99). Wie OSCS induziert das höher sulfatierte Dextransulfat im Polymer-Assay eine stärkere Fluoreszenintensität (DexS-Standard und DexS-inkubation) als Heparin (UFH-Standard).

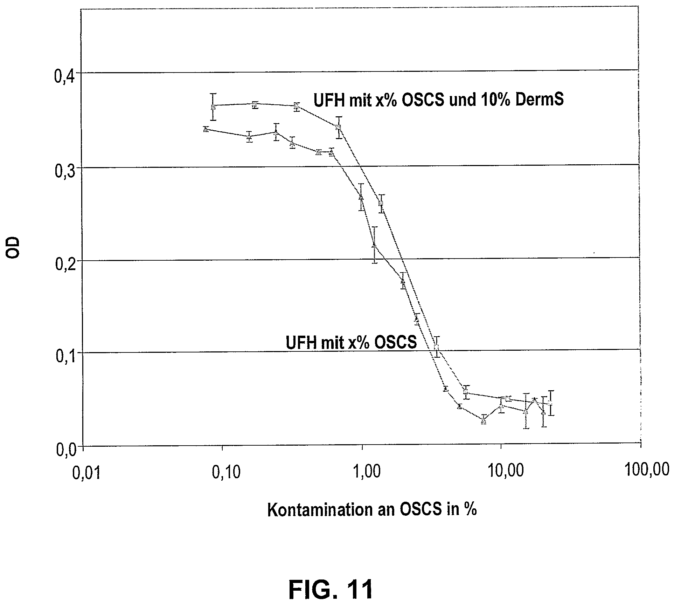

- Fig. 11: zeigt aXa-Assay-Ergebnisse von OSCS-Heparin-Mischungen mit und ohne 10% Dermatansulfat nach Inkubation (100 min) mit 1,5 SU/ml Heparinase.

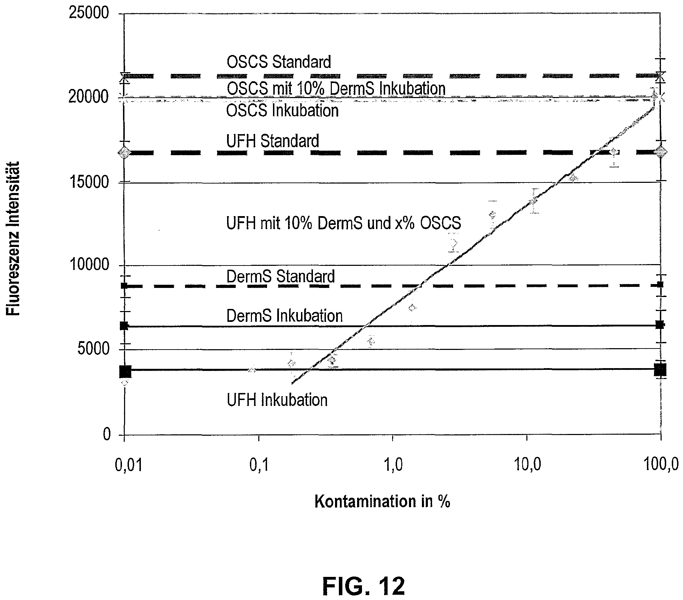

- Fig. 12: zeigt Fluoreszenztest-Ergebnisse von OSCS-Heparin-Mischungen mit einem Gehalt von 10% Dermatansulfat nach Inkubation (100 min) mit 1,5 SU/ml Heparinase. Die Zunahme der Fluoreszenzintensität in Abhängigkeit von der prozentualen Kontamination des Heparins mit OSCS wird durch die zusätzliche Verunreinigung mit 10% Dermatansulfat nicht beeinflusst (R² = 0,97).

### Beispiel

### SCHRITT 1: HEPARINABBAU MIT HEPARINASE

Die zu untersuchenden Heparin-Proben werden in 0,9%iger NaCl-Lösung zu 100 µg/ml gelöst. Für die Kalibrierung werden OSCS-Heparin-Standards hergestellt, die jeweils einen definierten %-Anteil an OSCS enthalten. Ihr OSCS-Gehalt sollte so gewählt werden, dass die Proben innerhalb dieses Bereiches liegen (z.B. 8 Standards im Bereich von 0,1 - 10 % OSCS). Das Heparin der Standards sollte den Proben entsprechend gewählt werden, d.h. UFH oder NMH. Zusätzlich werden 100 µg/ml Heparin, OSCS und ggf. Dermatansulfat als Kontrollen inkubiert.

In die Wells einer Mikrotiterplatte (MTP) werden jeweils 13 µl der Proben, der OSCS-Heparin-Standards, reinen Heparins und reinen OSCSs (jeweils 100 µg/ml) und 13 µl 0,9 %-iger NaCl-Lösung als Kontrolle (Heparinase-Kontrolle) pipettiert. Es werden jeweils zwei Wells mit der gleichen Lösung belegt.

Die Heparinase-Stammlösung wird mit Heparinase-Puffer verdünnt und auf eine Aktivität von 1,5 SU/ml eingestellt. Von dieser werden 117 µl zu den vorgelegten Proben pipettiert, so dass die Substrat-Konzentration 10 µg/ml beträgt. Zur Durchmischung wird die MTP 1 min bei 600 RPM geschüttelt.

Anschließend werden aus allen Wells 10 µl abpipettiert und sofort eingefroren. Diese Aliquots dienen zur Überprüfung des Abbaus durch die Heparinase im aXa-Assay (0 min-Werte). Durch die Temperaturerniedrigung wird ein Abbau durch die Heparinase gehemmt.

Es folgt eine Inkubation von 100 min bei 37 °C unter Schütteln (600 RPM). Nach Ablauf der Inkubationszeit werden wiederum 10 µl für den aXa-Assay und 45 µl für den Polymer-H-Test abpipettiert. Die abgenommenen Aliquots werden für mindestens 30 min bei -20 °C eingefroren, da dadurch der Abbau durch die Heparinase gestoppt wird.

Nach dem Auftauen werden die 10 µl-Aliquots für den aXa-Assay 1:20 mit 190 µl TRIS-Puffer verdünnt und 1 min (600 RPM) geschüttelt. Hiervon werden jeweils 25 µl als Untersuchungslösung (UL) für den aXa-Assay in die Wells einer MTP pipettiert, wobei jeweils Doppelwerte bestimmt werden (somit insgesamt 4 Werte pro Heparin-Probe, Standard etc.).

Für den Polymer-Test werden jeweils Aliquots von 45 µl der aufgetauten Lösungen in die Wells einer MTP pipettiert (ebenfalls Doppelwerte) und mit 135 µl 0,9 % NaCl verdünnt.

Beide Tests werden dann wie weiter unten beschrieben durchgeführt.

Während der Testentwicklung wurden die verschiedensten Parameter des Heparinabbaus mit Heparinase (u.a. Inkubationszeit, Abstoppen des Abbaus, Enzym- und Substratkonzentration) überprüft und optimiert. Die Kontrolle des Abbaus erfolgte jeweils anhand der Bestimmung der aXa-Aktivität von Heparin, die im Laufe der Inkubation abnimmt. Fig. 5 zeigt exemplarisch die aXa-Aktivität von Heparin zu Beginn und nach einer Inkubation von 100 min mit unterschiedlichen Heparinasekonzentrationen. Als endgültige Parameter wurden eine Inkubationszeit von 100 min sowie eine Heparinasekonzentration von 1,5 SU/ml und eine Heparinkonzentration von 100 µg/ml festgelegt (jeweils Pipettierkonzentration). Unter diesen Bedingungen findet ein vollständiger und reproduzierbarer Abbau statt.

Setzt man zu viel Heparinase oder zu wenig Heparin ein, wird schon während des 1-minütigen Schüttelns so viel Heparin abgebaut, dass eine Bestimmung zum Startpunkt der Inkubation einen Abbau im aXa-Test anzeigt. Unter dem Aspekt der Zeit ist ein schneller Abbau zwar günstig, aber die Heparinasemenge darf nur soweit gesteigert werden, dass OSCS sie noch hemmen kann. Dies kann jedoch genutzt werden, wenn eine Quantifizierung von OSCS in hochgradig verunreinigten Proben gewünscht ist. Bei Einsatz von mehr Substrat wird die Amplitude des aXa-Assays geringer, da auch Kontaminanten freies Heparin dann nicht vollständig abgebaut wird. Eine Hemmung durch gegebenenfalls enthaltenes OSCS kann zwar angezeigt werden, aber der Nachweis ist weniger empfindlich und die Quantifizierung von OSCS wird ungenauer.

Fig. 5 zeigt die aXa-Aktivität von Heparin zu Beginn und nach 100 min Inkubation mit Heparinase. Die angegebenen Konzentrationen entsprechen den jeweiligen Pipettierkonzentrationen; die Heparinkonzentration im Ansatz lag somit bei 10 µg/ml. OSCS besitzt keine aXa-Aktivität, die "optical density" (OD) entspricht ungefähr der der ungehemmten FXa-Aktivität (Max-Wert). Bei Verwendung von 1,5 SU/ml Heparinase (Pipettierkonzentration) war der Heparinabbau optimal. Beim 0 min-Wert (nach 1 min Schütteln) war die aXa-Aktivität noch voll vorhanden, nach 100 min ergab sich reproduzierbar ein nahezu vollständiger Abbau. Insgesamt ergaben sich für 1,5 SU/ml Heparinase bessere Resultate als für 1,0 SU/ml.

### SCHRITT 2A: ANTI-FAKTOR Xa-ASSAY

Wie oben beschrieben werden jeweils 25 µl aller mit Heparinase inkubierten und anschließend mit TRIS-Puffer verdünnten Untersuchungslösung (UL) (0 min- und 100 min-Wert) in die Wells einer MTP pipettiert. Ferner wird eine Heparin-Standardkurve (0,025-0,5 µg/ml) zur Qualitätssicherung mitgeführt sowie ein Max-Wert (25 µl TRIS-Puffer statt UL) und ein Blank-Wert (25 µl aqua bidest statt FXa).

Nacheinander werden folgende Reagenzien in die Wells pipettiert:
1. 25 µl AT (0,1 U) in TRIS-Puffer → 5 min Inkubation bei RT
2. 25 µl FXa (3,55 nkat/ml) in Aqua bidest. → 2 min Inkubation bei 37 °C
3.50 µl S-2222 (25 mg / 33,7 ml) Aqua bidest. → 30 sec Schütteln im Optima® bei 37 °C,
dann Messung der OD bei 405 nm. Es werden Doppelwerte bestimmt. Die Auswertung erfolgt anhand der Blank-Wert-bereinigten OD.

### SCHRITT 2B: POLYMER-ASSAY

Wie oben beschrieben werden jeweils 45 µl der 100 min mit Heparinase inkubierten UL plus 135 µl 0,9% NaCl in die Wells einer Maxisorp^{®}-MTP pipettiert.

Zur Qualitätssicherung werden zusätzlich folgende Kontrollen mitgetestet: jeweils 180 µl einer Heparin-Standardreihe (d.h. reines UFH oder NMH) in den Konzentrationen 0 - 5 µg/ml zur Überprüfung der Linearität des Assays sowie jeweils 180 µl der zu untersuchenden Heparin-Proben (2,5 µg/ml in 0,9 % NaCl). Zur Ermittlung des Blank-Wertes (d.h. Eigenfluoreszenz von Polymer-H) werden 180 µL 0,9 % NaCl verwendet.

Nach Zugabe von jeweils 20 µl der Polymer-H-Lösung (0,075 mg/ml) wird die MTP ca. 5 min geschüttelt, dann 5 min bei RT im Optima^{®} inkubiert, nochmals 30 sec geschüttelt und von oben vermessen (λₑₓ 320-10nm, λₑₘ 510-10nm). Es werden Doppelwerte bestimmt. Die Auswertung erfolgt anhand der Blank-Wert-bereinigten Fluoreszenzintensitäten.

### ALTERNATIVE: HEPARINASE-POLYMER-ASSAY

Soll nicht gleichzeitig neben OSCS auch eine Verunreinigung mit Dermatansulfat erfasst werden, kann entweder nur der aXa-Assay (z.B. im Kliniklabor, wo der aXa-Assay etabliert ist) oder nur der Polymer-Assay durchgeführt werden. Im Fall einer OSCS-Bestimmung mittels Polymer-Assay bietet sich die Option eines automatisierbaren "ein-MTP-Schnelltests".

Beispielhaft besteht dieser aus folgenden Schritten:
1. Vorlegen von 5 µl UL in Maxisorp^{®}-MTP
2. Zugabe von 45 µl Heparinase-Lösung
3. Plazieren in Novostar^{®} zum Schütteln und Inkubieren
4. Automatisierte Zugabe von 150 µl Polymer-1-Lösung (0,01 mg/ml) (Titratorfunktion)
5. Schütteln, Inkubieren und Vermessen

Zusätzlich kann die Durchführung des Polymer-Assays durch Modifikation der Heparinasekonzentration und Inkubationszeit beschleunigt werden.

Im Folgenden sind 3 Beispiele zum Nachweis von Verunreinigungen in Heparin dargestellt. Beispiel 1 zeigt die Empfindlichkeit des Testsystems anhand von Heparin, das unterschiedliche Konzentrationen an OSCS enthält. Beispiel 2 demonstriert, dass auch andere potentielle Heparinfälschungen mit der Erfindung nachzuweisen sind. Beispiel 3 veranschaulicht, dass sich eine zusätzliche Verunreinigung mit Dermatansulfat durch die Kombination von aXa-Assay und Polymer-Assay festzustellen ist.

### Beispiel 1

Um den Messbereich und die Empfindlichkeit des aXa- und Fluoreszenztests zu bestimmen, wurden 16 Heparin-OSCS-Mischungen mit folgendem %-Gehalt an OSCS hergestellt:
0,078 - 0,163 - 0,250 - 0,325 - 0,500 - 0,625 - 1,00 - 1,25 - 2,00 - 2,50 - 4,00 - 5,00 - 7,50 -10,0 - 15,0 - 20,0 % OSCS (= Uk16 - Uk1)

Nach 100minütiger Inkubation mit 1,5 SU/ml Heparinase wurden diese wie oben beschrieben in den beiden Assays getestet. Fig. 6 veranschaulicht, dass die aXa-Aktivität von reinem Heparin durch den enzymatischen Abbau fast vollständig verschwindet, während die von OSCS-haltigem Heparin mehr oder weniger erhalten bleibt. Dieser Hemmeffekt von OSCS bildet die Grundlage für die Quantifizierung von OSCS im aXa-Assay, während er im Polymer-Assay entscheidend für dessen Empfindlichkeit ist.

Die OD von OSCS bleibt gleich und entspricht ungefähr der des MAX-Wertes, d.h. keine Hemmung der FXa-Aktivität. Die aXa-Aktivität von UFH zu Beginn wird durch den enzymatischen Abbau fast vollständig zerstört, die von UFH, das mit OSCS kontaminiert ist, jedoch nur partiell.

Im aXa-Test kommt es ab einer Konzentration von 0,3 % OSCS zu einer Abnahme der OD im Vergleich zu reinem Heparin (Fig. 7). Im Bereich von 0,5 - 5,0 % OSCS lässt sich der OSCS-Gehalt direkt bestimmen, bei höherer Kontamination kann, sofern eine Quantifizierung erwünscht ist, die Probe mit reinem Heparin vor der Inkubation mit Heparinase verdünnt werden. Die Abnahme der OD signalisiert eine Abnahme der FXa-Aktivität im Test bzw. eine Zunahme der aXa-Aktivität der Probe und beruht auf der Hemmung des Abbaus von Heparin durch die Heparinase.

Die Ergebnisse im Fluoreszenztest zeigen ab einem OSCS-Gehalt von 0,325 % eine mit zunehmender OSCS-Kontamination steigende Fluoreszenzintensität des Polymerkomplexes (Fig. 8). Sie ist linear zum Logarithmus des OSCS-Gehaltes und erlaubt eine Quantifizierung im Bereich von 0,625 - 10 % OSCS, bei höherer Kontamination kann, sofern eine Quantifizierung erwünscht ist, die Probe verdünnt oder die Heparinasemenge modifiziert werden. Die Fluoreszenzzunahme beruht teils auf OSCS, teils auf nicht abgebautem Heparin.

Fig. 8 zeigt die Fluoreszenztest-Ergebnisse von OSCS-Heparin-Mischungen nach Inkubation (100 min) mit 1,5 SU/ml Heparinase. Zunahme der Fluoreszenzintensität in Abhängigkeit von der prozentualen Kontamination des Heparins mit OSCS (R² = 0,97).

### Beispiel 2

Exemplarisch veranschaulichen Fig. 9 und 10, dass auch andere Verunreinigungen von Heparin mit dem erfundenen Test nachweisbar sind, da jegliche Kontamination, die den enzymatischen Abbau von Heparin hemmen, sowohl im aXa- als auch im Fluoreszenztest sichtbar werden.

Das partialsynthetisch hergestellte Dextransulfat, das wie OSCS, mit den üblichen Tests nicht nachweisbar ist und auch im NMR-Spektrum kein "OSCS-Signal" zeigt, führt wie OSCS zu einer konzentrationsabhängig abnehmenden OD im aXa-Assay (Fig. 9). Da das eingesetzte Dextransulfat die Heparinase stärker hemmt als OSCS, reagiert der aXa-Assay empfindlicher und es ist auch ein Gehalt < 0,5% quantitativ bestimmbar.

Analog reagiert auch der Fluoreszenztest etwas empfindlicher auf Dextransulfat als auf OSCS.

Fig. 10 zeigt die Fluoreszenztest-Ergebnisse von Dextransulfat-Heparin-Mischungen nach Inkubation (100 min) mit 1,5 SU/ml Heparinase. Die Fluoreszenzintensität nimmt in Abhängigkeit von der prozentualen Kontamination des Heparins mit DexS (R² = 0,99) zu. Wie OSCS induziert das höher sulfatierte Dextransulfat im Polymer-Assay eine stärkere Fluoreszenintensität (DexS-Standard und DexS-Inkubation) als Heparin (UFH-Standard).

### Beispiel 3

In Abhängigkeit vom Ausgangsmaterial (d.h. Schweinedarmmukosa), dem Isolations- und Aufreinigungsverfahren kann Heparin bis zu > 10% Dermatansulfat (= Chondroitinsulfat B) und auch andere Chrondroitinsulfate enthalten. Um die Robustheit des erfundenen Tests zu überprüfen, wurden OSCS-Heparin-Mischungen hergestellt, die zusätzlich 10% Dermatansulfat (DermS) enthielten.

Im aXa-Test nimmt die OD des doppelt kontaminierten Heparins mit steigendem OSCS-Gehalt ab, allerdings sind die OD-Werte etwas höher als die von OSCS-Heparin ohne Dermatansulfat (Fig. 11). Dies beruht darauf, dass Dermatansulfat keine aXa-Aktivität besitzt und somit die aXa-Aktivität von nicht abgebautem Heparin "verdünnt". Weiteren Tests zufolge besitzt Dermatansulfat keine hemmenden Effekte auf den Abbau von Heparin durch Heparinase.

Fig. 11 zeigt die aXa-Test-Ergebnisse von OSCS-Heparin-Mischungen mit und ohne 10% Dermatansulfat nach Inkubation (100 min) mit 1,5 SU/ml Heparinase.

Fig. 12 zeigt die Fluoreszenztest-Ergebnisse von OSCS-Heparin-Mischungen mit einem Gehalt von 10 % Dermatansulfat nach Inkubation (100 min) mit 1,5 SU/ml Heparinase. Die Zunahme der Fluoreszenzintensität in Abhängigkeit von der prozentualen Kontamination des Heparins mit OSCS wird durch die zusätzliche Verunreinigung mit 10 % Dermatansulfat nicht beeinflusst (R² = 0,97).

Im Fluoreszenztest zeigt sich, dass eine gleichzeitige Verunreinigung mit 10 % Dermatansulfat (ebenso wie mit 10 % Chondroitinsulfat) keinen Einfluss auf den Nachweis von OSCS hat (Fig. 12). Der Polymer-Assay ist demzufolge ein robuster Test zur quantitativen Bestimmung von OSCS und anderen den enzymatischen Abbau von Heparin hemmenden Verunreinigungen.

Wird für die Kalibrierung des aXa- und Polymer-Assays ein nahezu Dermatansulfat-freies Heparin benutzt, ergibt sich für ein Heparin, das sowohl mit OSCS als auch mit Dermatansulfat verunreinigt wird, im aXa-Assay ein scheinbar geringerer OSCS-Gehalt als im Polymer-Assay. Überschreitet die Differenz einen bestimmten Wert, ist dies ein Hinweis auf eine übermäßige Verunreinigung mit Dermatansulfat.

## Patentansprüche

1. Verfahren zur Bestimmung von Kontaminierungen von Heparin mit anderen sulfatierten Glykanen, mit den Schritten:
- Inkubieren einer vorbestimmten Menge Heparin mit einer vorbestimmten Menge eines Heparin spezifisch verdauenden Enzyms für eine vorbestimmte Zeitdauer, wobei die Menge des Enzyms in etwa der Menge Enzym entspricht, die innerhalb der vorbestimmten Zeitdauer eine in etwa identische Menge Kontaminanten freies Heparin vollständig abbauen würde, oder größer ist,
- Inkubieren des verdauten Heparins mit einem bei Vorliegen sulfatierter Glykane seine Fluoreszenzeigenschaften ändernden Indikatormolekül,
- Erfassen der Fluoreszenz des mit dem Indikatormolekül inkubierten, verdauten Heparins,
wobei das Vorliegen einer sulfatierte Glykane anzeigenden Fluoreszenz das Vorliegen von Kontaminanten anzeigt und wobei die Aktivität des spezifisch Heparin abbauenden Enzyms mittels einer Kontaminanten freies Heparin enthaltenden Standardprobe vor und nach Inkubation mit dem Enzym mittels des bei Vorliegen sulfatierter Glykane seine Fluoreszenzeigenschaften ändernden Indikatormoleküls kontrolliert wird.

2. Verfahren nach Anspruch 1, **dadurch gekennzeichnet, dass** das spezifisch Heparin verdauende Enzym Heparinase ist.

3. Verfahren nach Anspruch 2, **dadurch gekennzeichnet, dass** die Heparinase Heparinlyase I (EC 4.2.2.7) ist.

4. Verfahren nach einem der vorhergehenden Ansprüche, **dadurch gekennzeichnet, dass** das Indikatormolekül 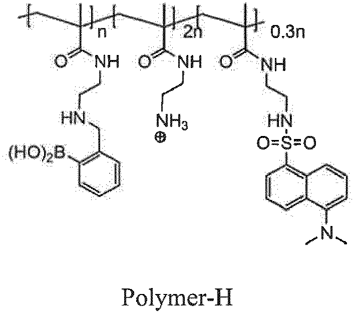 ist.

5. Verfahren nach einem der vorhergehenden Ansprüche, **gekennzeichnet durch** den weiteren Schritt: Bestimmen der Anti-Faktor-Xa-Aktvität des verdauten Heparins, wobei Vorliegen einer sulfatierte Glykane anzeigenden Fluoreszenz und das Vorliegen einer Anti-Faktor-Xa-Aktvität das Vorliegen von übersulfatiertem Chondroitinsulfat (OSCS) und/oder Dextransulfat und/oder einem anderen das Heparin spezifisch verdauende Enzym hemmenden sulfatierten Glykan anzeigt.

6. Verfahren nach einem der vorhergehenden Ansprüche, **gekennzeichnet durch** Bestimmen der Menge der im Heparin enthaltenen Kontaminanten anhand von Kalibrierwerten, die mit Kontaminanten freiem Heparin hergestellt wurden, dem unterschiedlichen Mengen an Kontaminanten beigemengt wurde.

7. Verfahren nach Anspruch 6, **dadurch gekennzeichnet, dass** eine einen vorbestimmten Wert überschreitende Differenz zwischen der mit dem Indikatormolekül bestimmten Konzentration der Kontaminanten von der durch die Anti-Faktor-Xa-Aktvität ermittelten Konzentration der Kontaminanten das Vorliegen von Dermatansulfat- und/oder Chondroitinsulfat und/oder Proteinen anzeigt.

8. Verfahren nach einem der vorhergehenden Ansprüche, **dadurch gekennzeichnet, dass** als Lösungsmittel eine wässrige 0,9 % NaCL-Lösung verwendet wird.

9. Verfahren nach einem der vorhergehenden Ansprüche, **dadurch gekennzeichnet, dass** das Heparin Bestandteil eines pharmazeutischen Präparats ist.

## Claims

1. A method for determining contaminations of heparin with other sulfated glycans, comprising the steps of:
- incubating a predetermined amount of heparin with a predetermined amount of a specific heparin-digesting enzyme for a predetermined time period, wherein the amount of the enzyme approximately corresponds to the amount of enzyme which would entirely degrade an approximately identical amount of contaminants-free heparin in the predetermined time period, or is higher,
- incubating the digested heparin with an indicator molecule changing its fluorescence characteristics in the presence of sulfated glycans,
- detecting the fluorescence of the digested heparin incubated with the indicator molecule,
wherein the presence of a fluorescence indicative for sulfated glycans indicates the presence of contaminants and wherein the activity of the specific heparin-degrading enzyme is controlled by means of a contaminants-free heparin-containing standard sample before and after incubation with the enzyme by means of the indicator molecule changing its fluorescence characteristics in the presence of sulfated glycans.

2. The method according to claim 1, **characterized in that** the specific heparin-digesting enzyme is heparinase.

3. The method according to claim 2, **characterized in that** the heparinase is heparin lyase I (EC 4.2.2.7).

4. The method according to any one, of the preceding claims, **characterized in that** the indicator molecule is

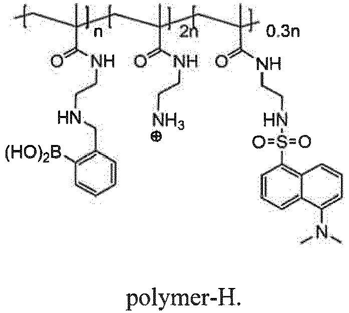

5. The method according to any one of the preceding claims, **characterized by** the further step of: determining the anti-factor-Xa-activity of the digested heparin, wherein the presence of a fluorescence indicative for sulfated glycans and the presence of an anti-factor-Xa-activity indicates the presence of oversulfated chondroitin sulfate (OSCS) and/or dextran sulfate and/or of another sulfated glycan inhibiting the specific heparin-digesting enzyme.

6. The method according to any one of the preceding claims, **characterized by** determining the amount of the contaminants contained in the heparin based on calibration values which have been prepared with contaminants-free heparin mixed with different amounts of contaminants.

7. The method according to claim 6, **characterized in that** a difference exceeding a predetermined value between the concentration of the contaminants determined by the indicator molecule and the concentration of the contaminants determined by the anti-faktor-Xa-activity indicates the presence of dermatan sulfate- and/or chondroitin sulfate and/or proteins.

8. The method according to any one of the preceding claims, **characterized in that** an aqueous 0.9 % NaCL solution is used as solvent.

9. The method according to any one of the preceding claims, **characterized in that** the heparin is an ingredient of a pharmaceutical preparation.

## Revendications

1. Procédé de détermination des contaminations de l'héparine par d'autres glycanes sulfatés, avec les étapes suivantes consistant à:
- incuber une quantité prédéterminée d'héparine avec une quantité prédéterminée d'une enzyme digérant spécifiquement l'héparine pendant une durée prédéterminée, dans lequel la quantité de l'enzyme correspond à peu près à la quantité de l'enzyme qui digérerait entièrement une quantité à peu près identique d'héparine exempte de contaminants dans la durée prédéterminée, ou est supérieure,
- incuber l'héparine digérée avec une molécule indicatrice modifiant ses propriétés de fluorescence en cas de présence de glycanes sulfatés,
- détecter la fluorescence de l'héparine digérée, incubée avec la molécule indicatrice,
dans lequel la présence d'une fluorescence indicatrice des glycanes sulfatés indique la présence de contaminants et dans lequel l'activité de l'enzyme dégradant spécifiquement l'héparine est contrôlée au moyen d'un échantillon étalon contenant une héparine exempte de contaminants avant et après l'incubation avec l'enzyme au moyen de la molécule indicatrice modifiant ses propriétés de fluorescence en cas de présence de glycanes sulfatés.

2. Procédé selon la revendication 1, **caractérisé en ce que** l'enzyme digérant spécifiquement l'héparine est l'héparinase.

3. Procédé selon la revendication 2, **caractérisé en ce que** l'héparinase est l'héparine lyase 1 (EC 4.2.2.7).

4. Procédé selon l'une des revendications précédentes, **caractérisé en ce que** la molécule indicatrice est le

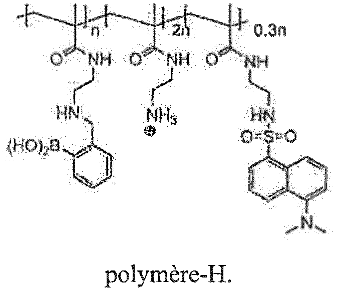

5. Procédé selon l'une des revendications précédentes, **caractérisé par** l'autre étape suivante consistant à: déterminer l'activité anti-facteur Xa de l'héparine digérée, dans lequel la présence d'une fluorescence indicatrice de glycanes sulfatés et la présence d'une activité anti-facteur Xa indiquent la présence de sulfate de chondroïtine hypersulfaté (OSCS) et/ou de sulfate de dextrane et/ou d'un autre glycane sulfaté inhibant l'enzyme digérant spécifiquement l'héparine.

6. Procédé selon l'une des revendications précédentes, **caractérisé par** la détermination de la quantité de contaminants contenus dans l'héparine à l'aide de valeurs de calibration qui ont été produites avec de l'héparine exempte de contaminants à laquelle différentes quantités de contaminants ont été ajoutées.

7. Procédé selon la revendication 6, **caractérisé en ce qu'**une différence dépassant une valeur prédéterminée entre la concentration en contaminants déterminée avec la molécule indicatrice et la concentration en contaminants établie par l'activité anti-facteur Xa indique la présence de sulfate de dermatane et/ou de sulfate de chondroïtine et/ou de protéines.

8. Procédé selon l'une des revendications précédentes, **caractérisé en ce qu'**une solution aqueuse à 0,9 % de NaCl est utilisée en tant que solvant.

9. Procédé selon l'une des revendications précédentes, **caractérisé en ce que** l'héparine fait partie d'une préparation pharmaceutique.
